# EUROPÄISCHE PATENTSCHRIFT

(11) **EP 2 301 453 B1**
(45) Veröffentlichungstag und Bekanntmachung des Hinweises auf die Patenterteilung: **15.07.2015**
(21) Anmeldenummer: 10009171.9
(22) Anmeldetag: 03.09.2010
(51) Int. Cl.: A61B 17/32, A61B 18/14

(54) **Medizinischer Resektor**
Medical resector
Dispositif de résection médical

(30) Priorität: 17.09.2009 DE 102009041602
(43) Veröffentlichungstag der Anmeldung: 30.03.2011
(73) Patentinhaber: Karl Storz GmbH & Co. KG, 78532 Tuttlingen (DE)
(72) Erfinder: Hamou, Jacques, Dr., 75015 Paris (FR); Simmen, Markus, 8603 Schwerzenbach (CH); Stillhard, Ottmar, 8266 Steckborn (CH)

(56) Entgegenhaltungen:
- WO-A1-93/16755
- DE-A1- 3 313 325
- DE-U1- 9 420 821
- US-A- 6 032 673
- US-A1- 2005 228 403

## Beschreibung

Die vorliegende Erfindung bezieht sich auf einen Resektor für medizinische Anwendungen mit einer rotierbaren Hochfrequenz-Elektrode.

In der Hochfrequenz-Chirurgie (HF-Chirurgie) wird zum Schneiden von Gewebe eine mit hochfrequenter elektrischer Leistung versorgte Elektrode verwendet. Zum Abtragen von Gewebe wird beispielsweise eine Hochfrequenz-Elektrode aus einer Drahtschlinge verwendet. Die Schlinge wird vom Operateur durch das Gewebe geschoben oder gezogen, um Gewebeschnipsel oder Gewebespäne nahezu beliebiger Länge herauszuschneiden.

Ein medizinischer Resektor im Sinne der folgenden Beschreibung kann mit einem Endoskop zu einem Resektoskop kombiniert werden, wobei ein Schaft des Endoskops in einem Kanal im Schaft des medizinischen Resektors angeordnet ist.

Ein von Jacques Hamou entwickelter medizinischer Resektor weist eine rotierende Drahtschlinge als Hochfrequenz-Elektrode auf. Die Drahtschlinge trägt das Gewebe in Form kleiner Späne bzw. Schnipsel ab, ähnlich beispielsweise der Funktion eines Fräskopfes zur spanabhebenden Bearbeitung von Werkstücken. Derartige medizinische Resektoren sind beispielsweise in der WO 2006/048199 A1 und der DE 10 2006 039 696 A1 beschrieben. Die Resektion bzw. die Ablation bzw. der Abtrag des Gewebes in kleinen Stücken ermöglicht eine unmittelbare Sichtkontrolle, eine vereinfachte Handhabung und einen saubereren und genaueren Abtrag von Gewebe. Außerdem können die kleinen Gewebeteile leicht entfernt werden, beispielsweise durch eine in den medizinischen Resektor integrierte Absaugung.

In der WO 93/16755 A ist ein Ablationskatheter zur Behandlung von Herzrhythmusstörungen beschrieben. Der Katheter umfasst einen vergleichsweise steifen rohrförmigen Abschnitt und einen flexiblen und bewegbaren distalen Abschnitt. Am distalen Ende des Katheters ist eine Ablationselektrode vorgesehen, die mittels eines Drahts vom proximalen Ende des Katheters aus rotiert werden kann.

In der US 6,032,373 ist eine Vorrichtung zum Entfernen von Gewebe mit einem starren Schaft und einer rotierbaren Elektrode am distalen Ende des Schafts beschrieben.

In der DE 33 13 325 A1 ist ein Operationsinstrument mit einem drehbar gelagerten und hochfrequenzgespeisten Resektionselement beschrieben. In einem Führungsrohr ist ein zum Führungsrohr koaxial gelagertes Antriebsrohr vorgesehen, an dessen vorderem Ende das Resektionselement befestigt ist.

Wie bei allen für mikroinvasive medizinische Eingriffe verwendeten Geräten ist auch bei einem medizinischen Resektor ein möglichst geringer Schaftdurchmesser wünschenswert. Der Schaft eines medizinischen Resektors weist in der Regel einen Kanal zum Einführen des Schafts eines Endoskops auf. Durch das Endoskop kann der Abtrag von Gewebe mittels des medizinischen Resektors unmittelbar beobachtet werden. Ferner weist der Schaft eines medizinischen Resektors mit einer rotierbaren Hochfrequenz-Elektrode eine Welle zum Übertragen der Rotationsbewegung von einer Antriebseinrichtung zur rotierbaren Hochfrequenz-Elektrode auf. Aufgrund des möglichst geringen Querschnitts des Schafts des medizinischen Resektors liegt die Welle dicht neben dem Endoskop bzw. dem Kanal für das Endoskop. Hinzu kommt, dass die rotierbare Hochfrequenz-Elektrode nicht über die Kontur des Schafts des medizinischen Resektors überstehen sollte, um beispielsweise ein Einführen mittels eines Trokars zu ermöglichen. Um gleichzeitig bei gegebenem Durchmesser des Schafts des medizinischen Resektors einen maximalen Durchmesser der Hochfrequenz-Elektrode zu ermöglichen, muss die Welle möglichst nah an der Symmetrieachse des Schafts liegen. Dabei entstehen jedoch am proximalen Ende geometrische Probleme bei der Kopplung des medizinischen Resektors und einer Antriebseinrichtung zum Rotieren der rotierbaren Hochfrequenz-Elektrode.

Weitere Probleme bei herkömmlichen medizinischen Resektoren mit rotierender Hochfrequenz-Elektrode sind die Spülung des Arbeitsbereichs und die Entfernung von abgetrennten Gewebestücken aus demselben. Die Spülung und die Entfernung von abgetrennten Gewebestücken sind erforderlich, um eine klare Sicht, beispielsweise durch das erwähnte Endoskop, auf den Arbeitsbereich zu ermöglichen.

Weitere bei herkömmlichen medizinischen Resektoren mit rotierender Hochfrequenz-Elektrode oft nicht oder nur unzureichend gelöste Probleme sind der Schutz der Patienten vor Verletzung durch die rotierbare Hochfrequenz-Elektrode und der Schutz der rotierbaren Hochfrequenz-Elektrode vor Beschädigung, insbesondere während des Einführens und Heranführens des medizinischen Resektors an den Arbeitsbereich in einem Hohlraum im Körper des Patienten.

Eine Aufgabe der vorliegenden Erfindung besteht darin, einen verbesserten medizinischen Resektor zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, bei einem medizinischen Resektor zum Abtragen, Schneiden oder Koagulieren von menschlichem oder tierischem Gewebe mit einer rotierbaren Hochfrequenz-Elektrode am distalen Ende eines geraden Schafts und einer starren Welle, die vom proximalen Ende zum distalen Ende des Schafts verläuft und am distalen Ende mit der Hochfrequenz-Elektrode mechanisch gekoppelt ist, die Welle am distalen Ende des Schafts in der Mitte oder möglichst nah an der Mitte des Querschnitts des Schafts und am proximalen Ende des Schafts am oder möglichst nah am Rand des Querschnitts des Schafts anzuordnen.

Die Welle ist starr. Die starre Welle weist eine einzige gerade Rotationsachse auf. Die Rotationsachse verläuft so, dass der Abstand der Rotationsachse von der Mitte des Schafts des medizinischen Resektors vom proximalen Ende zum distalen Ende des Schafts streng monoton abnimmt. Sowohl die Welle als auch der Schaft des medizinischen Resektors sind gerade, wobei die Rotationsachse der Welle nicht-parallel zur Längsachse des Schafts angeordnet ist. Diese Nicht-Parallelität umfasst eine Abweichung von der Parallelität, die über die in der Medizintechnik übliche Präzision bzw. über die in der Medizintechnik üblichen Toleranzen hinausgeht. Insbesondere liegt zwischen der Längsachse des medizinischen Resektors bzw. seines Schafts einerseits und der Rotationsachse der Welle ein Winkel von mindestens 0,4°, insbesondere ein Winkel zwischen 0,6° und 1,0° vor. Die Längsachse des medizinischen Resektors ist insbesondere die Längsachse des Schafts des medizinischen Resektors.

Die Längsachse des Schafts die Gerade, auf der die Mittelpunkte der Querschnittsflächen des Schafts liegen.

Der medizinische Resektor ist beispielsweise ein Hysteroablator oder ein Uroablator.

Diese Nicht-Parallelität bzw. dieser Winkel zwischen der Rotationsachse der Welle und der Längsachse des Schafts des medizinischen Resektor ermöglicht gleichzeitig eine zentrale oder fast zentrale Anordnung der rotierbaren Hochfrequenz-Elektrode am distalen Ende einerseits und eine exzentrische Anordnung des Endes der Welle und der daran angeordneten Kupplungseinrichtung am proximalen Ende des medizinischen Resektors andererseits.

Die zentrale Anordnung der rotierbaren Hochfrequenz-Elektrode am distalen Ende des Schafts kann unter anderem deshalb vorteilhaft sein, weil die Hochfrequenz-Elektrode damit einen maximalen Durchmesser haben kann, ohne über die Kontur des Schafts des medizinischen Resektors überzustehen. Die exzentrische Anordnung der Welle und der Kupplungseinrichtung am proximalen Ende ermöglicht insbesondere einen maximalen Abstand beispielsweise von einem in den Schaft des medizinischen Resektors einführbaren Schaft eines Endoskops und damit maximalen Bauraum für eine Antriebseinrichtung und die Kopplung des medizinischen Resektors mit der Antriebseinrichtung.

Mit einer zentralen Anordnung der rotierbaren Hochfrequenz-Elektrode am distalen Ende des Schafts des medizinischen Resektors ist eine möglichst zentrale Anordnung gemeint, soweit diese unter den gegebenen Bedingungen möglich ist. Diese genau zentrale Anordnung ist nur unter bestimmten Bedingungen möglich. Wenn beispielsweise im Schaft des Resektors ein Raumbereich, insbesondere ein abgetrennter Kanal, zum Einführen eines Schafts eines Endoskops vorgesehen ist, kann die Rotationsachse der Welle nur dann im Zentrum des Schafts des medizinischen Resektors liegen, wenn das Zentrum des Schafts nicht innerhalb des Kanals oder zu nah an dessen Rand liegt. Dabei wird die Größe des Kanals in erster Linie durch den Durchmesser des in den Kanal einzuführenden Endoskops bestimmt. Deshalb kann die Rotationsachse der Welle nur dann im Zentrum des Schafts liegen, wenn die Summe des Durchmessers des Endoskops, des Radius der Welle und gegebenenfalls der Dicke einer Zwischenwand zwischen Endoskop und Welle kleiner als der Innenradius des Schafts des medizinischen Resektors ist.

Anderenfalls ist der Abstand zwischen der Rotationsachse der Welle einerseits und dem Zentrum des Querschnitts des Schafts des medizinischen Resektors andererseits mindestens die Differenz zwischen der Summe aus dem Durchmesser des Schafts des Endoskops, des Radius der Welle und gegebenenfalls der Dicke der Trennwand zwischen Endoskop und Welle einerseits und dem Innenradius des Schafts des medizinischen Resektors andererseits. Ferner muss berücksichtigt werden, dass für ein reibungsarmes Einführen des Endoskops in den dafür vorgesehenen Kanal im Schaft des medizinischen Resektors das Lumen dieses Kanals etwas größer sein muss als der Querschnitt des Endoskops. Im Fall eines nicht-kreisförmigen Querschnitts des Schafts des medizinischen Resektors oder eines nicht-kreisförmigen Querschnitts des Schafts des Endoskops gilt Entsprechendes.

Am proximalen Ende des Schafts des medizinischen Resektors ist die Welle insbesondere am Rand des Querschnitts des Schafts angeordnet. Die Welle kann am proximalen Ende des Schafts nur so nah am Rand des Querschnitts angeordnet werden, dass sie diesen nicht berührt sondern einen für eine reibungsarme Rotation erforderlichen Abstand aufweist. Der erforderliche Abstand der Welle vom Rand des Querschnitts des Schafts wird gegebenenfalls durch die Wanddicke eines Führungsrohrs oder eines Lagers für die Welle vergrößert. Der Abstand der Rotationsachse der Welle vom Rand des Lumens des Schafts des medizinischen Resektors muss im Wesentlichen nur um den Betrag eines für eine reibungsarme Rotation der Welle erforderlichen Abstands größer sein als die Summe des Radius der Welle und gegebenenfalls der Wanddicke eines Führungsrohrs der Welle oder eines Lagers für die Welle. Anders ausgedrückt ist, um am proximalen Ende des medizinischen Resektors einen maximalen Abstand der Welle von einem in den Schaft des medizinischen Resektors eingeführten Endoskop zu erzielen, die Welle am proximalen Ende des Schafts insbesondere unmittelbar angrenzend an die innere Oberfläche des Schafts des medizinischen Resektors oder von dieser nur getrennt durch ein Lager oder die Wandung eines Führungsrohrs angeordnet.

Wenn ein medizinischer Resektor, wie er oben beschrieben wurde, einen Schaft mit einer kreiszylindrischen Mantelfläche aufweist, kann die rotierbare Hochfrequenz-Elektrode eine Drahtschleife umfassen oder aus einer Drahtschleife bestehen, deren Mitte, deren Flächenmittelpunkt oder deren Rotationsachse auf oder zumindest so nah wie möglich an der Symmetrieachse der Mantelfläche des Schafts liegt. Diese Anordnung ist wiederum in der oben beschriebenen Weise durch die Durchmesser bzw. Radien des Schafts des medizinischen Resektors, des Schafts des Endoskops und der Welle begrenzt. Eine Anordnung des Flächenmittelpunkts der Drahtschleife an der Symmetrieachse oder möglichst nah an der Symmetrieachse der Mantelfläche des Schafts ermöglicht einen maximalen Durchmesser der rotierbaren Hochfrequenz-Elektrode ohne Überstand über die Kontur des Schafts des medizinischen Resektors. Insbesondere kann in diesem Fall der Durchmesser der rotierbaren Hochfrequenz-Elektrode so groß oder fast so groß sein wie der Außendurchmesser des Schafts des medizinischen Resektors.

Ein medizinischer Resektor, wie er oben beschrieben ist, kann eine Antriebseinrichtung umfassen oder für die Verwendung mit einer Antriebseinrichtung ausgebildet sein, die eine Drehfrequenz in einem Drehfrequenzbereich, der in einem Bereich von 10 Umdrehungen pro Minute bis 200 Umdrehungen pro Minute enthalten ist, bereitstellt.

Zur Ermittlung geeigneter Drehfrequenzen bzw. Drehzahlen bzw. Rotationsfrequenzen wurden zunächst theoretische Überlegungen und Berechnungen angestellt, die von Erfahrungswerten mit herkömmlichen, nicht rotierenden schlingenförmigen Hochfrequenz-Elektroden ausgehen. Für derartige herkömmliche Hochfrequenz-Elektroden kann abhängig vom Gewebe und anderen Umständen des Einzelfalls eine Schnittgeschwindigkeit von ca. 10 Millimeter pro Sekunde als bewährt angesehen werden. Es wird davon ausgegangen, dass die Schnittgeschwindigkeit an keiner Stelle an der rotierenden Hochfrequenz-Elektrode über der bewährten Schnittgeschwindigkeit von 10 Millimeter pro Sekunde liegen soll.

Die Schnittgeschwindigkeit ist außer von der Drehfrequenz auch vom Radius linear abhängig. Berechnungen ergeben bei einer maximalen Schnittgeschwindigkeit von 10 Millimetern pro Sekunde und bei einem Durchmesser der Drahtschlinge bzw. der Hochfrequenz-Elektrode von 4 Millimetern eine Drehfrequenz von 48 Umdrehungen pro Minute, bei 6 Millimetern eine Drehfrequenz von 32 Umdrehungen pro Minute und bei 8 Millimetern eine Drehfrequenz von 24 Umdrehungen pro Minute. Umfangreiche, detaillierte und deshalb auch langwierige experimentelle Untersuchen durch Jacques Hamou bestätigen, dass bei den üblichen Durchmessern der Hochfrequenz-Elektrode bzw. der Drahtschlinge bei Drehfrequenzen zwischen 30 Umdrehungen pro Minute und 60 Umdrehungen pro Minute überraschend nicht nur hinsichtlich der Schnittleistung und der Schnittqualität erhebliche Vorteile erzielt werden. Abhängig von der Größe und Form der Hochfrequenz-Elektrode sowie vom zu schneidenden Gewebe sind auch niedrigere Drehfrequenzen ab etwa 10 Umdrehungen pro Minute oder 20 Umdrehungen pro Minute und höhere Drehfrequenzen bis ca. 100 Umdrehungen pro Minute, im Einzelfall bis 150 Umdrehungen pro Minute oder 200 Umdrehungen pro Minute, vorteilhaft.

Überraschend liegt bei den genannten Drehfrequenzen, insbesondere bei Drehfrequenzen zwischen 30 Umdrehungen pro Minute und 60 Umdrehungen pro Minute, ein Optimum vor hinsichtlich der Abtragsleistung einerseits sowie der Beobachtbarkeit und Kontrollierbarkeit des Abtrags andererseits. Trotz einer guten Abtragleistung sind die abgetragenen Gewebestücke so klein, dass sie die Sicht durch ein beispielsweise im medizinischen Resektor angeordnetes Endoskop auf den Arbeitsbereich nur unwesentlich beeinträchtigen. Die geringe Beeinträchtigung der Sicht durch das Endoskop rührt auch daher, dass die Gewebestücke schnell und ohne das Risiko einer Verstopfung des medizinischen Resektors abgesaugt und damit aus dem Blickfeld entfernt werden können.

Überraschend spielt die Drehfrequenz der rotierbaren Hochfrequenz-Elektrode auch für die Belastung des medizinischen Personals und damit wiederum indirekt für die Qualität des erzielten Ergebnisses eine wichtige Rolle. Bei den genannten Drehfrequenzen, besonders bei Drehfrequenzen bis 60 Umdrehungen pro Minute, kann die Bewegung der Hochfrequenz-Elektrode vom Auge des medizinischen Personals noch ohne Weiteres wahrgenommen und aufgelöst werden. Die Beobachtung der Hochfrequenz-Elektrode wird deshalb noch nicht als anstrengend wahrgenommen. Jedes einzelne abgetragene Gewebestück und die dadurch freigelegten tieferen Gewebeschichten und deren Oberflächen können verfolgt bzw. beobachtet werden. Bei deutlich höheren Drehfrequenzen über 100 Umdrehungen pro Minute und noch mehr bei über 200 Umdrehungen pro Minute wirkt die Bewegung der Hochfrequenz-Elektrode und der Gewebestücke und ihre Beobachtung zunehmend ermüdend. Dies ist bei medizinischen Anwendungen nicht nur in Rücksicht auf das medizinische Personal sondern vor allem auch auf die Patienten gefährlich.

Ferner ist bei den genannten Drehfrequenzen, insbesondere im Bereich von 30 Umdrehungen pro Minute bis 60 Umdrehungen pro Minute, der Abtrag besonders gut kontrollierbar. Die beschriebene gute Beobachtbarkeit einerseits und der ausreichend schnelle aber nicht zu schnelle Abtrag sind offenbar besonders geeignet für eine Steuerung und Regelung des Abtrags durch medizinisches Personal.

Die genannten Vorteile werden so und vor allem auch in dieser Kombination bei keiner anderen Drehfrequenz erzielt. Niedrigere Drehfrequenzen haben eine unzureichende Abtragsleistung zur Folge. Außerdem werden die einzelnen abgetragenen Gewebestücke zu groß, um einwandfrei abgesaugt zu werden. Ferner verdecken die Gewebestücke die freigelegten Gewebeschichten zu lange bevor sie ganz abgelöst und beispielsweise durch Absaugen aus dem Blickfeld entfernt sind.

Bei höheren Drehfrequenzen über 200 Umdrehungen pro Minute, je nach Gewebe oft schon bei Drehfrequenzen über 100 Umdrehungen pro Minute oder über 60 Umdrehungen pro Minute, ist die Qualität der Schnitte, insbesondere die Koagulation, oft unzureichend. Hinzu kommt, dass die Beobachtung der schnellen Bewegungen für das Auge des medizinischen Personals ermüdend ist. Je nach Gewebe können ferner die abgetragenen Gewebestücke zu klein sein, wodurch sie insbesondere an Oberflächen haften und nur noch schwer abzusaugen sind.

Ausführungsformen der vorliegenden Erfindung beruhen auf der Idee, eine Krageneinrichtung zur Anbringung an einem distalen Ende eines Schafts eines medizinischen Resektors mit einer rotierbaren Hochfrequenz-Elektrode zum Abtragen, Schneiden oder Koagulieren von menschlichem oder tierischem Gewebe so auszubilden, dass sie die rotierbare Hochfrequenz-Elektrode teilweise umgibt, wenn sie am distalen Ende des Schafts des medizinischen Resektors angeordnet ist. Um die rotierbare Hochfrequenz-Elektrode bzw. den von ihr eingenommenen Raumbereich teilweise mantelförmig zu umgeben, steht die Krageneinrichtung insbesondere distal über das Ende des Schafts des medizinischen Resektors über. Die Krageneinrichtung kann damit in vielen Fällen die rotierbare Hochfrequenz-Elektrode vor einer unerwünschten Berührung und einen Patienten vor einer Verletzung durch die rotierbare Hochfrequenz-Elektrode schützen.

Insbesondere weist die Krageneinrichtung die Form eines Ausschnitts aus einem Zylindermantel auf. Dabei kann die Form der Krageneinrichtung im Wesentlichen der Mantelfläche des Schafts des medizinischen Resektors, für den die Krageneinrichtung vorgesehen und ausgebildet ist, entsprechen. Die Krageneinrichtung kann dazu ausgebildet sein, teilweise an der Mantelfläche des Schafts des medizinischen Resektors anzuliegen und mit dieser verschraubt, verklebt, verrastet, verschweißt, verlötet oder mit ihr durch Klemmung oder mittels eines - insbesondere in die Krageneinrichtung integrierten - Bügels verbunden zu sein. Die Krageneinrichtung kann damit so ausgebildet sein, dass sie die Kontur des Schafts des medizinischen Resektors nicht oder nur unwesentlich erweitert. Die Verwendbarkeit mit einem herkömmlichen Trokar kann damit erhalten bleiben.

Insbesondere überlappt ein proximaler Randbereich der Krageneinrichtung mit einem distalen Randbereich der die Mantelfläche bildenden Wand des Schafts.

Im Bereich des Überlapps kann die Krageneinrichtung außerhalb der Wand des Schafts angeordnet sein. Ein Vorteil dieser Anordnung kann darin bestehen, dass die Hochfrequenz-Elektrode genauso groß ausgebildet werden kann wie ohne die Krageneinrichtung. Alternativ kann die Krageneinrichtung im Bereich des Überlapps innerhalb der Wand des Schafts angeordnet sein. Dadurch kann die Krageneinrichtung ohne Weiteres vollständig innerhalb der Kontur des Schafts liegen. Der medizinische Resektor kann damit beispielsweise mit einem Trokar verwendbar sein, mit dem auch ein entsprechender medizinischer Resektor ohne die Krageneinrichtung verwendbar ist.

Sowohl bei Anordnung der Krageneinrichtung außerhalb als auch bei Anordnung der Krageneinrichtung innerhalb der Wand des Schafts kann die Wand des Schafts eine einfache Geometrie aufweisen, die mit geringem Aufwand erzeugt werden kann. Insbesondere kann die Krageneinrichtung außen oder innen an der distal kragenförmig und mit konstanter Wandstärke überstehenden Wand eines herkömmlichen Schafts angeordnet sein. Alternativ kann die Wand des Schafts im Bereich des Überlapps mit der Krageneinrichtung eine reduzierte Wandstärke aufweisen. Auch die Krageneinrichtung kann im Bereich des Überlapps eine reduzierte Wandstärke aufweisen.

Ein weiterer Vorteil der Krageneinrichtung besteht darin, dass sie ausgestaltet sein kann, um am distalen Ende des Schafts des medizinischen Resektors austretende Spülflüssigkeit zu leiten und damit die Spülung des Arbeitsbereichs zu verbessern. Die verbesserte Spülung kann auch einen verbesserten Abtransport von durch den medizinischen Resektor abgetragenen Gewebestücken und damit eine verbesserte Sicht auf das bearbeitete Gewebe und die bearbeitete Gewebeoberfläche zur Folge haben.

Ferner kann die Krageneinrichtung an einem medizinischen Resektor eine Verbesserung der Absaugung von abgetragenen Gewebestücken durch den Schaft des medizinischen Resektors bewirken. Auch dadurch kann der Abtransport der abgetragenen Gewebestücke und der Blick auf den Arbeitsbereich verbessert werden.

Um die genannten Vorteile hinsichtlich der Spülung und der Absaugung zu erzielen, kann die Krageneinrichtung ausgebildet sein, um die Hochfrequenz-Elektrode bezogen auf die Längsachse des Schafts des medizinischen Resektors zu mindestens der Hälfte zu umgeben. Dabei ist die Krageneinrichtung insbesondere ausgebildet, um die Hochfrequenz-Elektrode bezogen auf die Längsachse des Schafts zu höchstens drei Vierteln zu umgeben. Ein Winkel von ca. 250° hat sich besonders bewährt.

Eine Krageneinrichtung, wie sie oben beschrieben ist, kann ferner so ausgebildet sein, dass bei Anordnung der Krageneinrichtung am distalen Ende des Schafts eines medizinischen Resektors das distale Ende der Hochfrequenz-Elektrode des medizinischen Resektors gegenüber einem distalen Rand der Krageneinrichtung übersteht oder beide bündig abschließen. Alternativ kann die Krageneinrichtung ausgebildet sein, dass bei Anordnung der Krageneinrichtung am distalen Ende des Schafts eines medizinischen Resektors der distale Rand der Krageneinrichtung gegenüber einem distalen Ende der Hochfrequenz-Elektrode des medizinischen Resektors übersteht.

Die drei genannten Konfigurationen können für unterschiedliche Anwendungen vorteilhaft sein. Je länger die Krageneinrichtung ist und je weiter sie über die Hochfrequenz-Elektrode übersteht, desto besser schützt sie diese vor einer unerwünschten Berührung und Beschädigung und den Patienten vor einer Verletzung durch die Hochfrequenz-Elektrode. Je kürzer die Krageneinrichtung ausgebildet ist und je weiter die Hochfrequenz-Elektrode gegenüber der Krageneinrichtung übersteht, desto besser können auch Oberflächen mit dem medizinischen Resektor bearbeitet werden, die senkrecht zur Längsachse des Schafts des medizinischen Resektors liegen.

Die Krageneinrichtung kann ferner die Funktion einer Führung beim Abtrag von Gewebe übernehmen. Beispielsweise wird der medizinische Resektor so geführt, dass der distale Rand der Krageneinrichtung an der noch nicht abgetragenen Oberfläche oder an der durch Abtrag von Gewebe neu gebildeten Oberfläche anliegt. Beides ermöglicht eine gute Steuerung er Dicke der abgetragenen Schicht. Die Dicke der abgetragenen Schicht kann dabei vom Überstand der rotierbare Hochfrequenz-Elektrode gegenüber dem distalen Rand der Krageneinrichtung bzw. vom Überstand des distalen Rands der Krageneinrichtung gegenüber der rotierbaren Hochfrequenz-Elektrode sowie vom Winkel zwischen dem Schaft des medizinischen Resektors und der zu bearbeitenden oder bearbeiteten Oberfläche abhängen.

Für viele Anwendungen hat sich eine Konfiguration als vorteilhaft bewährt, bei der das distale Ende der Hochfrequenz-Elektrode und der distale Rand der Krageneinrichtung bündig abschließen, das heißt im Wesentlichen in einer zur Längsachse des Schafts des medizinischen Resektors senkrechten Ebene liegen.

Um die Wirkung der über die Hochfrequenz-Elektrode übertragenen Hochfrequenz-Leistung auf das menschliche oder tierische Gewebe, insbesondere die schneidende, abtragende und/oder koagulierende Wirkung, nicht zu beeinträchtigen, kann die Krageneinrichtung Polyetheretherketon (PEEK), einen anderen Kunststoff, Keramik oder ein anderes elektrisch isolierendes Material aufweisen.

Ein medizinischer Resektor, wie er oben beschrieben ist, kann eine Krageneinrichtung, wie sie oben beschrieben ist, aufweisen. Ferner kann ein medizinische Resektor, wie er oben beschrieben ist, mit einem Endoskop zu einem Resektoskop kombiniert werden, wobei ein Schaft des Endoskops in einem Kanal im Schaft des medizinischen Resektors angeordnet ist.

Ausführungsformen der vorliegenden Erfindung beruhen auf der Idee, am distalen Ende eines medizinischen Resektors eine Absaugöffnung vorzusehen, die gegenüber der Längsachse des Schafts des medizinischen Resektors geneigt ist.

Die Absaugöffnung weist einen Rand auf, der in einer Ebene oder in einer gekrümmten Fläche liegen kann. Als Fläche der Absaugöffnung wird nachfolgend diejenige Fläche bezeichnet, in der der Rand der Absaugöffnung liegt, und deren sämtliche Schnittlinien mit Ebenen senkrecht zur Längsachse des Schafts des medizinischen Resektors gerade sind. Die Absaugöffnung ist insofern gegenüber der Längsachse des Schafts des medizinischen Resektors geneigt, als die mittlere bzw. gemittelte Flächennormale der Fläche der Absaugöffnung nicht parallel zur Längsachse des Schafts des medizinischen Resektors ist.

Insbesondere beträgt der Winkel zwischen der mittleren Flächennormalen der Fläche der Absaugöffnung und der Längsachse des Schafts des medizinischen Resektors mindestens 30 Grad. Als besonders geeignet haben sich Winkel zwischen 45 Grad und 75 Grad erwiesen.

Die Absaugöffnung ist insbesondere so ausgebildet, dass alle Flächennormalen der Fläche der Absaugöffnung mit der Längsachse des Schafts des medizinischen Resektors einen Winkel von mindestens 30 Grad einschließen.

Die beschriebene Neigung der Absaugöffnung kann eine wirksame Absaugung eines getrübten oder verunreinigten Fluids und/oder von Gewebestücken aus der Umgebung des distalen Endes des medizinischen Resektors unterstützen und verbessern. Die Absaugung wird insbesondere dadurch verbessert, dass die geneigte Absaugöffnung größer sein kann als es eine parallel zur Längsachse des Schafts des medizinischen Resektors ausgerichtete Absaugöffnung sein kann. Ferner kann die Absaugung dadurch verbessert werden, dass am distalen Ende des medizinischen Resektors - insbesondere am Rand eines distalen Endes eines Endoskops oder rund um das distale Ende des Endoskops - austretendes Spülfluid durch die Neigung der Absaugöffnung nicht unmittelbar sondern erst nach großräumigerer Mischung mit den abzusaugenden Verunreinigungen und/oder Gewebestücken abgesaugt wird. Dies kann eine schnelle und weitgehende Klärung des Fluids in einem Hohlraum, in dem der medizinische Resektor eingesetzt wird, fördern.

Ausführungsformen der vorliegenden Erfindung beruhen auf der Idee, am distalen Ende eines medizinischen Resektors eine Krageneinrichtung und eine Absaugöffnung vorzusehen, wobei ein Abschnitt des Rands der Absaugöffnung eine stetige Fortsetzung eines Abschnitts des Rands der Krageneinrichtung bildet.

Insbesondere bildet ein Abschnitt des Rands der Absaugöffnung eine stetige Fortsetzung eines Abschnitts des Rands der Krageneinrichtung, wenn ein Ende des Abschnitts des Rands der Absaugöffnung und ein Ende des Abschnitts des Rands der Krageneinrichtung einen Abstand aufweisen, der nicht größer als eine Wanddicke der Krageneinrichtung oder nicht größer als eine Wanddicke des Schafts des medizinischen Resektors oder nicht größer als die Summe der Wanddicke der Krageneinrichtung und der Wanddicke des Schafts des medizinischen Resektors ist.

Insbesondere bildet ein Abschnitt des Rands der Absaugöffnung eine stetige Fortsetzung eines Abschnitts des Rands der Krageneinrichtung, wenn an einander gegenüberliegenden Enden des Abschnitts des Rands der Absaugöffnung und des Rands der Krageneinrichtung die beiden Abschnitte Richtungen aufweisen, die sich um weniger als 30 Grad oder um weniger als 10 Grad unterscheiden.

Beispielsweise weist die Absaugöffnung einen im Wesentlichen D-förmigen Rand mit einem geraden Abschnitt und einem bogenförmigen Abschnitt auf. Der gerade Abschnitt des Rands der Absaugöffnung ist quer zur Längsachse des Schafts des medizinischen Resektors angeordnet. Ecken zwischen dem geraden Abschnitt und dem bogenförmigen Abschnitt des Rands der Absaugöffnung sind nahe gegenüberliegenden Abschnitten des Rands der Krageneinrichtung angeordnet. Die genannten gegenüberliegenden Abschnitte des Rands der Krageneinrichtung sind nahe den genannten Ecken des Rands der Absaugöffnung insbesondere im Wesentlichen parallel. Die genannten gegenüberliegenden Abschnitte des Rands der Krageneinrichtung sind nahe den genannten Ecken des Rands der Absaugöffnung insbesondere jeweils im Wesentlichen parallel zu dem jeweils benachbarten Ende des bogenförmigen Abschnitts des Rands der Absaugöffnung.

Die Ausbildung und Anordnung der Absaugöffnung derart, dass ein Abschnitt des Rands der Absaugöffnung eine stetige Fortsetzung eines Abschnitts des Rands der Krageneinrichtung bildet, kann bei geringem Platzbedarf eine besonders gute Absaugung ermöglichen. Insbesondere kann sie eine besonders große und/oder besonders dicht an die Krageneinrichtung heranreichende Absaugöffnung ermöglichen. Dadurch kann diese Ausbildung und Anordnung der Absaugöffnung gleichzeitig eine gute Leitung eines Spülfluids durch die Krageneinrichtung und eine wirksame Absaugung von Spülfluid ermöglichen, wobei aber am distalen Ende des medizinischen Resektors - insbesondere am Rand eines distalen Endes eines Endoskops oder rund um das distale Ende des Endoskops - austretendes Spülfluid überwiegend nicht unmittelbar, sondern erst nach großräumigerere Durchmischung mit zu entfernenden Substanzen durch die Absaugöffnung abgesaugt wird. Dies kann eine schnelle und weitgehende Klärung des Fluids in einem Hohlraum, in dem der medizinische Resektor eingesetzt wird, fördern.

Mit der beschriebenen Absaugöffnung können die Krageneinrichtung so ausgebildet sein und der medizinische Resektor bei der vorgesehenen Anwendung so geführt werden, dass abgetragene Gewebestücke und andere die Sicht beeinträchtigende Verunreinigungen durch der bearbeiteten Oberfläche gegenüberliegende Abschnitte des Rands der Krageneinrichtung zu der Absaugöffnung geführt und wirksam entfernt werden.

Ein medizinischer Resektor mit einer Absaugöffnung, die gegenüber der Längsachse des Schafts des medizinischen Resektors geneigt ist und/oder deren Rand einen Abschnitt aufweist, der eine stetige Fortsetzung eines Abschnitts des Rands einer Krageneinrichtung bildet, kann eine Welle aufweisen, deren Rotationsachse parallel oder, wie oben beschrieben, nicht parallel zur Längsachse des Schafts des medizinischen Resektors ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Resektors;
- Figur 2: eine schematische Darstellung eines weiteren medizinischen Resektors;
- Figur 3: eine schematische Darstellung eines weiteren medizinischen Resektors;
- Figur 4: schematische Darstellungen eines distalen Endes eines Schafts eines medizinischen Resektors und einer Krageneinrichtung;
- Figur 5: eine schematische Darstellung der Krageneinrichtung aus Figur 4;
- Figur 6: eine schematische Darstellung der Krageneinrichtung aus Figur 4;
- Figur 7: eine schematische Darstellung eines weiteren medizinischen Resektors;
- Figur 8: eine weitere schematische Darstellung des medizinischen Resektors aus Figur 7;
- Figur 9: eine weitere schematische Darstellung des medizinischen Resektors aus Figur 7.

### Beschreibung der Ausführungsformen

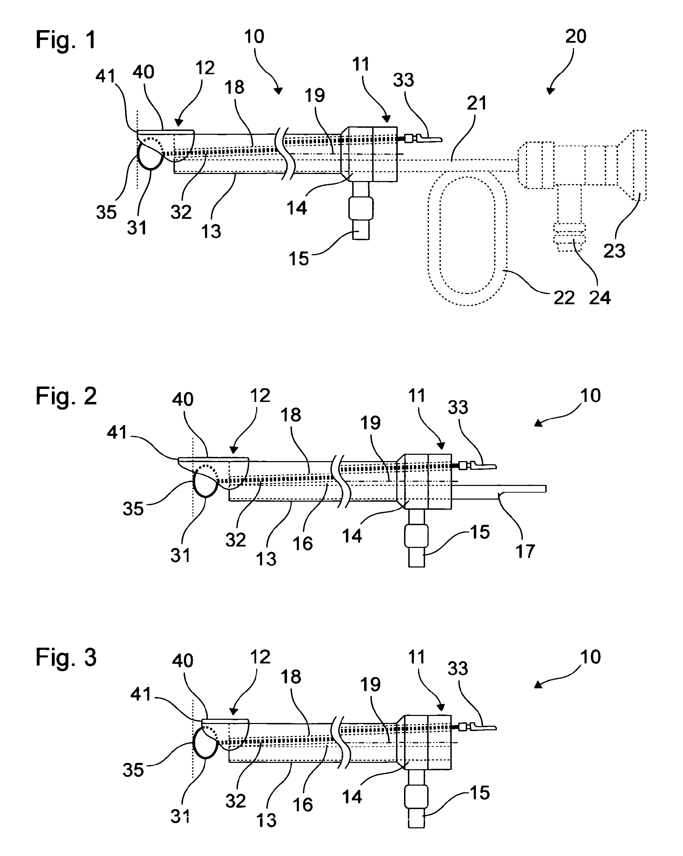

Figur 1 zeigt eine schematische Darstellung eines medizinischen Resektors 10 mit einem proximalen Ende 11, einem distalen Ende 12 und einem Schaft 13, der sich vom proximalen Ende 11 zum distalen Ende 12 erstreckt. Der Schaft 13 ist zylindrisch mit einem kreisförmigen, elliptischen, ovalen oder beliebigen anderen Querschnitt. Die Längsachse 19 des Schafts 13 ist parallel zur Zeichenebene der Figur 1. Da die Längsachse 19 nahe dem distalen Ende 12 des Resektors 10 im Bereich anderer in Figur 1 dargestellter Einrichtungen verläuft, ist sie nur nahe dem proximalen Ende 11 des medizinischen Resektors 10 dargestellt, um die Darstellung nicht zu überfrachten. Tatsächlich verläuft die Längsachse 19 entlang des gesamten medizinischen Resektors 10.

Am proximalen Ende 11 des medizinischen Resektors 10 weist dieser eine Abflusskammer 14 mit einem Anschluss 15 für eine in Figur 1 nicht dargestellte Saugeinrichtung auf. In die Abflusskammer 14 und den Schaft 13 kann ein Endoskop 20 bzw. dessen Schaft 21 eingeführt werden. Dazu kann der medizinische Resektor 10 einen entsprechenden Kanal mit Öffnungen an beiden Enden aufweisen, der sich durch die Abflusskammer 14 und den Schaft 13 vom proximalen Ende 11 bis zum distalen Ende 12 des medizinischen Resektors erstreckt. Ferner umfasst der medizinische Resektor 10 ein Führungsrohr 18 und/oder zwei oder mehr Lager für eine nachfolgend beschriebene Welle 32. Der medizinische Resektor 10 ist auch ohne das Endoskop 20 verwendbar. Das Endoskop 20 ist deshalb in gestrichelten Linien dargestellt.

Das Endoskop 20 umfasst den bereits erwähnten Schaft 21, einen die Handhabbarkeit des Endoskops 20 verbessernden Griff 22 oder eine andere Handhabe, ein Okular 23 und eine Kupplung 24 zur Verbindung des Endoskops 20 mit einer Lichtquelle, die in Figur 1 nicht dargestellt ist. Alternativ ist der Griff 22 oder eine andere Handhabe abweichend von der Darstellung in Figur 1 an einem Schaft angeordnet, in den der Schaft 21 des Endoskops eingeführt und dort insbesondere befestigt oder verriegelt werden kann. Mit dem Griff 22 oder einer anderen Handhabe am Endoskop 20 oder alternativ an einem Schaft, in dem der Schaft 21 des Endoskops 20 geführt und gehalten ist, kann das Endoskop 20 unabhängig von dem medizinischen Resektor 10 geführt, beispielsweise im medizinischen Resektor 10 gedreht werden.

Der medizinische Resektor 10 umfasst ferner eine rotierbare Hochfrequenz-Elektrode 31 an seinem distalen Ende 12. Die rotierbare Hochfrequenz-Elektrode 31 ist an einer Welle 32 befestigt oder mit dieser einstückig ausgeführt. Die Welle 32 erstreckt sich im Inneren des Schafts 13 des medizinischen Resektors 10 von der rotierbaren Hochfrequenz-Elektrode 31 am distalen Ende 12 bis zu einer Kupplung 33 am proximalen Ende 11 im Führungsrohr 18 des medizinischen Resektors und/oder in zwei oder mehr Lagern, die in Figur 1 nicht dargestellt sind. Die Kupplung 33 ist proximal der Abflusskammer 14 angeordnet. Die Welle 32 ist ausgebildet, um eine Rotation der Kupplung 33 zur Hochfrequenz-Elektrode 31 zu übertragen.

Die Welle 32 und damit auch die Rotationsachse der rotierbaren Hochfrequenz-Elektrode 31, der Welle 32 und der Kupplungseinrichtung 33 sind nicht parallel zur Längsachse 19 des Schafts 13. Die Rotationsachse und die Längsachse 19 bilden einen Winkel von mindestens 0,4°, insbesondere einen Winkel zwischen 0,6° und 1,0° oder einen Winkel von im Wesentlichen 0,8°. Dieser Winkel ermöglicht einerseits eine zentrale Anordnung der Hochfrequenz-Elektrode 31 am distalen Ende 12 und andererseits einen maximalen Abstand zwischen der Kupplungseinrichtung 33 am proximalen Ende 11 und dem Schaft 21 des Endoskops 20.

Am distalen Ende 12 ist eine optionale Krageneinrichtung 40 aus Polyetheretherketon, Kunststoff, Keramik oder einem anderen elektrisch isolierenden Material angeordnet. Die Krageneinrichtung 40 schützt die Hochfrequenz-Elektrode 31 vor einer unerwünschten Berührung und Beschädigung und den menschlichen oder tierischen Körper vor einer Verletzung durch die Hochfrequenz-Elektrode 31, insbesondere beim Einführen des medizinischen Resektors 10.

Ferner kann die Krageneinrichtung 40 eine am distalen Ende 12 des medizinischen Resektors 10 austretende Flüssigkeit leiten. Zusätzlich oder alternativ kann die Krageneinrichtung 40 eine am distalen Ende 12 des medizinischen Resektors 10 erzeugte Absaugwirkung auf den Raumbereich um die Hochfrequenz-Elektrode 31 kanalisieren oder konzentrieren. In beiden Fällen verbessert die Krageneinrichtung 40 den Blick durch das Endoskop 20 auf eine mittels der rotierbaren Hochfrequenz-Elektrode 31 bearbeitete Oberfläche.

Mit dem oben anhand der Figur 1 dargestellten medizinischen Resektor 10 ist eine Antriebseinrichtung für die rotierbare Hochfrequenz-Elektrode 31 koppelbar, die in Figur 1 nicht dargestellt ist. Die Antriebseinrichtung ist insbesondere als Antriebseinheit ausgebildet, die ohne eine Notwendigkeit einer zusätzlichen Getriebeeinheit eine Drehfrequenz in einem Drehfrequenzbereich bereitstellt, der im Bereich von 10 Umdrehungen pro Minute bis 200 Umdrehungen pro Minute enthalten ist.

Mit einer gestrichelten Linie senkrecht zur Längsachse 19 des Schafts 13 des medizinischen Resektors 10 am distalen Ende 35 der rotierbaren Hochfrequenz-Elektrode 31 und am distalen Rand 41 der Krageneinrichtung 40 wird verdeutlicht, dass beide im Wesentlichen in einer Ebene senkrecht zur Längsachse 19 des Schafts 13 des medizinischen Resektors 10 liegen.

Die Darstellungen in den Figuren 2 und 3 entsprechen weitgehend der Darstellung in Figur 1, wobei jedoch jeweils kein Endoskop im medizinischen Resektor 10 angeordnet ist. Im Unterschied zu der in Figur 1 dargestellten Konfiguration weist der in Figur 2 dargestellte medizinische Resektor 10 eine Krageneinrichtung 40 auf, deren distaler Rand 41 gegenüber dem distalen Ende 35 der rotierbaren Hochfrequenz-Elektrode 31 übersteht. Dies kann gegenüber der Konfiguration aus Figur 1 einen verbesserten Schutz der rotierbaren Hochfrequenz-Elektrode 31 vor einer Beschädigung bei einer unerwünschten Berührung und einen verbesserten Schutz eines Patienten vor einer Verletzung durch die rotierbare Hochfrequenz-Elektrode 31 bewirken.

Bei der in Figur 3 dargestellten Konfiguration steht das distale Ende 35 der rotierbaren Hochfrequenz-Elektrode 31 gegenüber dem distalen Rand 41 der Krageneinrichtung 40 über. Diese Konfiguration kann im Vergleich zu den oben anhand der Figuren 1 und 2 dargestellten Konfigurationen eine Bearbeitung von Oberflächen, die senkrecht oder im Wesentlichen senkrecht zur Längsachse 19 des Schafts 13 des medizinischen Resektors 10 liegen, erleichtern.

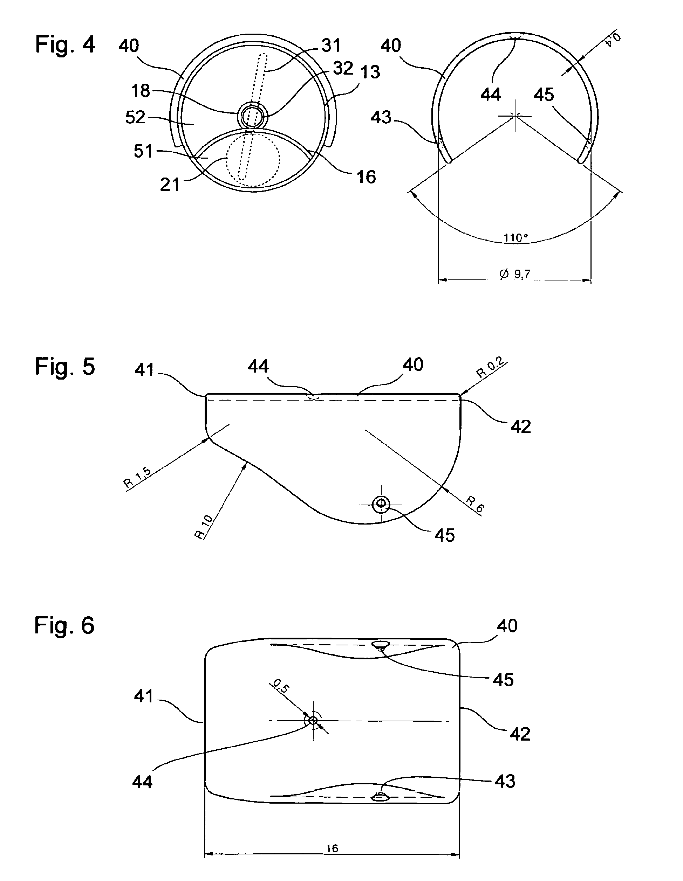

Die in den Figuren 2 und 3 dargestellten medizinischen Resektoren 10 unterscheiden sich von dem oben anhand der Figur 1 dargestellten ferner durch eine Zwischenwand 16, welche den Innenraum des Schafts 13 des medizinischen Resektors 10 in ein erstes Lumen und ein zweites Lumen unterteilt. Das erste Lumen ist für die Anordnung eines Schafts eines Endoskops darin und/oder zum Leiten eines Spülfluids zum distalen Ende 12 des Schafts 13 vorgesehen. Das zweite Lumen ist zum Absaugen von mittels der rotierbaren Hochfrequenz-Elektrode abgetrennten Gewebestücken vorgesehen. Die Zwischenwand kann wie in den Figuren 2 und 3 dargestellt rohrförmig sein oder den nachfolgend anhand der Figur 4 dargestellten Querschnitt aufweisen.

Der in Figur 2 dargestellte medizinische Resektor 10 unterscheidet sich von den oben anhand der Figuren 1 und 3 dargestellten ferner dadurch, dass proximal der Abflusskammer 14 ein Führungsrohr 17 vorgesehen ist. Das Führungsrohr 17 verlängert das durch die Zwischenwand 16 abgetrennte erste Lumen proximal. Der Schaft 21 eines Endoskops 20, wie es oben anhand der Figur 1 dargestellt wurde, kann in das Führungsrohr 17 und das durch die Zwischenwand 16 abgetrennte erste Lumen bis zum distalen Ende 12 des medizinischen Resektors 10 eingeführt werden. Die erwähnte, in den Figuren 1 bis 3 nicht dargestellte Antriebseinrichtung kann ferner an dem Führungsrohr 17 abgestützt und/oder befestigt werden.

Figur 4 zeigt eine schematische Draufsicht auf ein distales Ende eines Schafts eines medizinischen Resektors, wie er oben anhand der Figuren 1 bis 3 dargestellt wurde (links), 7,66und auf eine Krageneinrichtung alleine (rechts). Die Zeichenebene liegt jeweils senkrecht zur Längsachse 19 des Schafts 13.

In Figur 4 links ist erkennbar, dass die Zwischenwand 16 das Innere des Schafts 13 des medizinischen Resektors in ein erstes Lumen 51 und ein zweites Lumen 52 unterteilt. Im ersten Lumen 51 kann eine Spülflüssigkeit zum distalen Ende des Schafts 13 geführt werden und dort austreten. Alternativ oder zusätzlich kann im ersten Lumen 51 der Schaft 21 eines Endoskops 20 angeordnet sein oder werden, wie es oben anhand der Figur 1 dargestellt ist.

Im zweiten Lumen 52 ist die Welle 32 in einem Führungsrohr 18 oder in entsprechenden Lagern angeordnet. Ferner kann über das zweite Lumen 52 flüssiges, gasförmiges oder festes Material vom distalen Ende des Schafts 13 abgesaugt werden. Insbesondere können dadurch Gewebestücke aus dem Arbeitsbereich entfernt werden, die mittels der rotierbaren Hochfrequenz-Elektrode 31 abgetragen wurden.

In Figur 4 ist ferner erkennbar, dass die Krageneinrichtung 40 den Schaft 13 und die rotierbare Hochfrequenz-Elektrode 31 bezogen auf die Längsachse 19 des Schafts 13 zu mehr als der Hälfte, in diesem Fall zu ca. zwei Dritteln umschließt.

In Figur 4 rechts ist eine Krageneinrichtung 40 dargestellt, die sich von der in Figur 4 links dargestellten Krageneinrichtung in einigen Details unterscheidet. Insbesondere sind bei der in Figur 4 rechts dargestellten Krageneinrichtung 40 auch beispielhaft einige Abmessungen angegeben. Es ist unter anderem erkennbar, dass die in Figur 4 rechts dargestellte Krageneinrichtung 40 ausgebildet ist, um einen Schaft 13 und eine rotierbare Hochfrequenz-Elektrode 31 mit einem Gesamtwinkel von ca. 250° zu umschließen.

Ferner weist die in Figur 4 rechts dargestellte Krageneinrichtung drei Befestigungsöffnungen 43, 44, 45 auf. Jede dieser Befestigungsöffnungen 43, 44, 45 ist als Durchgangsloch mit einer teilweise konischen Form zur Aufnahme einer Senkkopfschraube ausgebildet. Mittels dreier Senkkopfschrauben in den drei Befestigungsöffnungen kann die Krageneinrichtung 40 am distalen Ende 12 eines Schafts 13 eines medizinischen Resektors befestigt werden. Alternativ kann die Krageneinrichtung 40 mittels einer anderen Anzahl von Senkkopf- oder anderen Schrauben und/oder durch Kleben, Löten, Schweißen, Verrasten oder Klemmen am distalen Ende 12 eines Schafts 13 eines medizinischen Resektors befestigt werden.

Die Figuren 5 und 6 zeigen weitere Darstellungen der in Figur 4 rechts dargestellten Krageneinrichtung 40. Figur 5 zeigt eine Seitenansicht, wobei die Zeichenebene der Figur 5 parallel zur Längsachse 19 des Schafts 13 des medizinischen Resektors 10 ist und den Zeichenebenen der Figuren 1 bis 3 entspricht. Figur 6 zeigt eine Unteransicht, wobei die Zeichenebene senkrecht zu der Zeichenebene der Figur 5 und parallel zur Längsachse 19 des Schafts 13 des medizinischen Resektors 10 ist. In beiden Figuren 5 und 6 sind ebenso wie in Figur 4, beispielhaft Abmessungen angegeben.

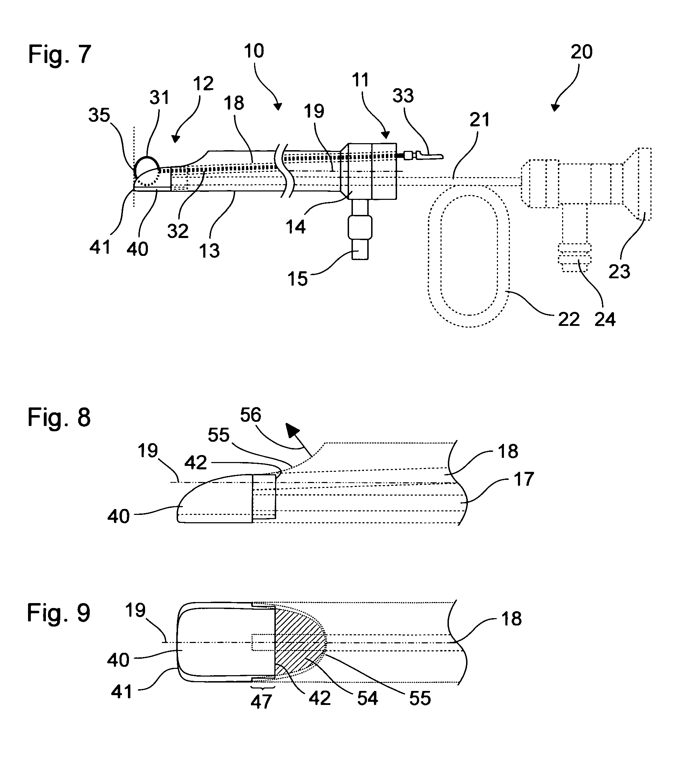

Figur 7 zeigt eine schematische Darstellung eines weiteren medizinischen Resektors 10, der in einigen Merkmalen den oben anhand der Figuren 1 bis 3 beschriebenen medizinischen Resektoren ähnelt. Insbesondere ist der medizinische Resektor 10 mit einem in Figur 7 gestrichelt dargestellten Endoskop 20 kombinierbar. Ferner weist der medizinische Resektor 10 ähnlich wie der oben anhand der Figur 1 dargestellte medizinische Resektor eine rotierbare Hochfrequenz-Elektrode 31 auf, deren distales Ende 35 mit dem distalen Rand 41 einer Krageneinrichtung 40 abschließt bzw. mit diesem in einer Ebene senkrecht zur Längsachse 19 des medizinischen Resektors liegt.

Abweichend von den oben anhand der Figuren 1 bis 3 dargestellten medizinischen Resektoren ist bei dem in Figur 7 gezeigten medizinischen Resektor 10 die Krageneinrichtung 40 bezogen auf die Welle 32, den Anschluss 15 und andere nicht zur Längsachse 19 symmetrische Merkmale umgekehrt angeordnet. Bezogen auf die nicht symmetrischen Merkmale des medizinischen Resektors umfasst die Krageneinrichtung 40 einen Bereich, der von der Krageneinrichtung des in Figur 1 dargestellten medizinischen Resektors nicht umfasst wird. Umgekehrt umfasst die Krageneinrichtung des in Figur 1 dargestellten medizinischen Resektors einen Bereich, der von der Krageneinrichtung 40 nicht umfasst wird.

Ähnlich wie bei den oben anhand der Figuren 1 bis 3 dargestellten medizinischen Resektoren überlappt die Krageneinrichtung 40 des in Figur gezeigten medizinischen Resektors mit dem distalen Ende des Schafts 13 bzw. der diesen bildenden Wandung. Abweichend von den oben anhand der Figuren 1 bis 3 dargestellten medizinischen Resektoren ist bei dem in Figur 7 gezeigten medizinischen Resektor 10 die Krageneinrichtung 40 im Überlappbereich nicht außerhalb des Schafts 13 sondern innerhalb bzw. an der Innenseite der Wandung des Schafts 13 angeordnet.

Ferner weist der Resektor 10 eine Absaugöffnung auf, die nachfolgend mit Bezug auf die Figuren 8 und 9 näher beschrieben wird. Die Figuren 8 und 9 zeigen schematische vergrößerte Darstellungen des distalen Endes des oben anhand der Figur 7 dargestellten medizinischen Resektors 10. Die Zeichenebene der Figur 8 ist parallel zur Längsachse 19 des medizinischen Resektors 10 entspricht der Zeichenebene der Figur 7. Die Zeichenebene der Figur 9 ist ebenfalls parallel zur Längsachse 19 des medizinischen Resektors, jedoch senkrecht zu den Zeichenebenen der Figuren 7 und 8. Um die Gestalt der Krageneinrichtung 40 klarer hervorzuheben, sind in den Figuren 8 und 9 die Krageneinrichtung 40 in durchgezogenen Linien und die Konturen des Schafts 13 des medizinischen Resektors 10 in fein unterbrochenen Linien dargestellt. Ferner sind das Führungsrohr 17 für ein Endoskop und das Führungsrohr 18 für die Welle des medizinischen Resektors 10 in unterbrochenen Linien angedeutet. Die Hochfrequenz-Elektrode und die Welle des medizinischen Resektors 10 sind in den Figuren 8 und 9 nicht dargestellt.

In den Figuren 8 und 9 ist die Überlappung der Krageneinrichtung 40 und der Wand des Schafts 13 in einem Bereich 47 erkennbar. Im Bereich 47 weist die Krageneinrichtung 40 eine reduzierte Wandstärke auf. Auch die Wand des Schafts 13 kann im Bereich 47 eine reduzierte Wandstärke aufweisen. Abweichend von der Darstellung in den Figur 7 bis 9 kann nur die Wand des Schafts 13 oder weder diese noch die Krageneinrichtung 40 im Bereich 47 eine reduzierte Wandstärke aufweisen. Im Bereich 47 sind die Krageneinrichtung 40 und die Wand des Schafts 13 beispielsweise mittels einer Klebung verbunden.

Am distalen Ende 12 weist der Schaft 13 eine Absaugöffnung 54 auf, die in Figur 9 schraffiert dargestellt ist, um sie herauszuheben. Die Absaugöffnugn 54 weist einen im Wesentlichen D-förmigen Rand 55 auf, der einen geraden und einen im Wesentlichen bogenförmigen Abschnitt umfasst. Der gerade Abschnitt des Rands 55 ist senkrecht zur Längsachse 19 des medizinischen Resektors 10. Ecken zwischen dem geraden Abschnitt und dem bogenförmigen Abschnitt des Rands 55 der Absaugöffnung 54 sind nahe dem Rand der Krageneinrichtung 40 angeordnet. Nahe diesen Ecken ist der bogenförmige Abschnitt des Rands 55 der Absaugöffnung 54 parallel oder im Wesentlichen parallel zum Rand der Krageneinrichtung 40.

Wie bereits oben dargestellt, wird als Fläche der Absaugöffnung 54 die Fläche bezeichnet, die vom Rand 55 der Absaugöffnung begrenzt wird, und deren sämtliche Schnittlinien mit Ebenen senkrecht zur Längsachse 19 des medizinischen Resektors 10 gerade sind. Bei dem anhand der Figuren 7 bis 9 dargestellten Resektor 10 sind alle lokalen Flächennormalen 56 der Fläche der Absaugöffnung parallel zu den Zeichenebenen der Figuren 7 und 8. Alle lokalen Flächennormalen 56 der Fläche der Absaugöffnung schließen mit der Längsachse 19 des medizinischen Resektors 10 Winkel ein, die im Bereich von ca. 30 Grad bis ca. 90 Grad enthalten sind. Die mittlere bzw. gemittelte Flächennormale der Fläche der Absaugöffnung schließt mit der Längsachse 19 des medizinischen Resektors 10 einen Winkel ein, der im Bereich von ca. 45 Grad bis ca. 60 Grad liegt.

### Bezugszeichen

- 10: medizinischer Resektor
- 11: proximales Ende des medizinischen Resektors 10
- 12: distales Ende des medizinischen Resektors 10
- 13: Schaft des medizinischen Resektors 10
- 14: Abflusskammer am proximalen Ende 11 des medizinischen Resektors 10
- 15: Anschluss für Saugeinrichtung
- 16: Zwischenwand im Schaft 13 des medizinischen Resektors 10
- 17: Führungsrohr für Endoskop 20
- 18: Führungsrohr für Welle 32
- 19: Längsachse des medizinischen Resektors 10
- 20: Endoskop
- 21: Schaft des Endoskops 20
- 22: Griff am Schaft 21
- 23: Okular des Endoskops
- 24: Kupplung zur Verbindung des Endoskops 20 mit einer Lichtquelle
- 31: rotierbare Hochfrequenz-Elektrode
- 32: Welle
- 33: Kupplungseinrichtung
- 35: distales Ende der rotierbaren Hochfrequenz-Elektrode 31
- 40: Krageneinrichtung
- 41: distaler Rand der Krageneinrichtung 40
- 42: proximaler Rand der Krageneinrichtung 40
- 43: erste Befestigungsöffnung
- 44: zweite Befestigungsöffnung
- 45: dritte Befestigungsöffnung
- 46: Rand der Krageneinrichtung 40
- 47: Bereich des Überlapps der Krageneinrichtung 40 mit dem Schaft 21
- 51: erstes Lumen für Spülflüssigkeit
- 52: zweites Lumen für Absaugung
- 54: Absaugöffnung am distalen Ende des zweiten Lumens 52
- 55: Rand der Absaugöffnung 54
- 56: lokale Flächennormale der Fläche der Absaugöffnung 54

## Patentansprüche

1. **Medizinischer Resektor** (10) zum Abtragen, Schneiden oder Koagulieren von menschlichem oder tierischem Gewebe mit:
einer rotierbaren **Hochfrequenz-Elektrode** (31) am distalen Ende (12) eines geraden Schafts (13) des medizinischen Resektors (10);
einer starren, geraden **Welle** (32) mit einer einzigen, geraden Rotationsachse, die vom proximalen Ende (11) zum distalen Ende (12) des Schafts (13) verläuft und am distalen Ende (12) mit der Hochfrequenz-Elektrode (31) mechanisch gekoppelt ist,
wobei die Welle (32) am distalen Ende (12) des Schafts (13) in der **Mitte** oder möglichst nah an der Mitte des Querschnitts des Schafts (13) und am proximalen Ende (11) des Schafts (13) am **Rand** oder möglichst nah am Rand des Querschnitts des Schafts (13) angeordnet ist und
die Rotationsachse so verläuft, dass der Abstand der Rotationsachse von der Mitte Querschnitts des Schafts (13) des medizinischen Resektors (10) vom proximalen Ende (11) zum distalen Ende (12) des Schafts streng monoton abnimmt.

2. Medizinischer Resektor (10) nach dem vorangehenden Anspruch, bei dem der Schaft (13) eine **kreiszylindrische** Mantelfläche aufweist,
die rotierbare Hochfrequenz-Elektrode (31) eine **Drahtschleife** umfasst, deren Mitte in oder an der Symmetrieachse der Mantelfläche des Schafts (13) liegt.

3. Medizinischer Resektor (10) nach einem der vorangehenden Ansprüche, ferner mit:
einem **Führungsrohr** (18), in dem die Welle (32) angeordnet ist, wobei das Führungsrohr (18) am proximalen Ende (11) des Schafts (13) an der Außenwand des Schafts (13) des medizinischen Resektors (10) angeordnet ist.

4. Medizinischer Resektor (10) nach einem der vorangehenden Ansprüche, ferner mit einer **Kupplungseinrichtung** (33) am proximalen Ende (11) des Schafts (13), zur lösbaren mechanischen Kopplung mit einer Antriebseinrichtung, die Drehfrequenzen in einem Drehfrequenzbereich bereitstellt, der im Bereich von **10** Umdrehungen pro Minute bis **200** Umdrehungen pro Minute enthalten ist.

5. Medizinscher Resektor (10) nach einem der vorangehenden Ansprüche, ferner mit:
einer **Krageneinrichtung** (40) zur Anordnung am distalen Ende (12) des Schafts (13) des medizinischen Resektors (10), wobei die Krageneinrichtung (40) dazu ausgebildet ist, die rotierbare Hochfrequenz-Elektrode (31) teilweise zu umgeben.

6. Medizinscher Resektor (10) nach dem vorangehenden Anspruch, bei dem die Krageneinrichtung (40) die Form eines Ausschnitts eines **Zylindermantels** aufweist.

7. Medizinscher Resektor (10) nach Anspruch 5 oder 6, bei dem die Krageneinrichtung (40) teilweise an der Mantelfläche des Schafts (13) des medizinischen Resektors (10) **anliegt**.

8. Medizinscher Resektor (10) nach einem der Ansprüche 5 bis 7, bei dem die Krageneinrichtung (40) die Hochfrequenz-Elektrode (31) bezogen auf die Längsachse (19) des Schafts (13) des medizinischen Resektors (10) zu **mindestens der Hälfte** umgibt.

9. Medizinscher Resektor (10) nach einem der Ansprüche 5 bis 8, bei dem die Krageneinrichtung (40) die Hochfrequenz-Elektrode (31) bezogen auf die Längsachse (19) des Schafts (13) zu **höchstens drei Vierteln** umgibt.

10. Medizinscher Resektor (10) nach einem der Ansprüche 5 bis 9, bei dem ein distales Ende (35) der Hochfrequenz-Elektrode (31) gegenüber einem distalen Rand (41) der Krageneinrichtung (40) **übersteht.**

11. Medizinscher Resektor (10) nach einem der Ansprüche 5 bis 9, bei dem ein distaler Rand (41) der Krageneinrichtung (40) mit einem distalen Ende (35) der Hochfrequenz-Elektrode (31) **bündig** abschließt.

12. Medizinscher Resektor (10) nach einem der Ansprüche 5 bis 9, bei dem ein distaler Rand (41) der Krageneinrichtung (40) gegenüber einem distalen Ende (35) der Hochfrequenz-Elektrode (31) **übersteht.**

13. Medizinischer Resektor (10) nach einem der vorangehenden Ansprüche, ferner mit:
einer **Absaugöffnung** (54) am distalen Ende (12) des medizinischen Resektors (10), wobei die Absaugöffnung (54) gegenüber der Längsachse (19) des medizinischen Resektors (10) **geneigt** ist.

14. Medizinischer Resektor (10) nach einem der Ansprüche 5 bis 12, ferner mit:
einer **Absaugöffnung** (54) am distalen Ende (12) des medizinischen Resektors (10), wobei ein Abschnitt des Rands (55) der Absaugöffnung (54) eine stetige Fortsetzung eines Abschnitts des Rands der Krageneinrichtung (40) bildet.

## Claims

1. Medical resector (10) for ablating, cutting or coagulating human or animal tissue, with:
a rotatable radiofrequency electrode (31) at the distal end (12) of a straight shank (13) of the medical resector (10);
a rigid, straight shaft (32) which has a single, straight rotation axis and which extends from the proximal end (11) to the distal end (12) of the shank (13) and, at the distal end (12), is mechanically coupled to the radiofrequency electrode (31),
wherein the shaft (32), at the distal end (12) of the shank (13), is arranged at the centre or as close as possible to the centre of the cross section of the shank (13), and, at the proximal end (11) of the shank (13), is arranged at the edge or as close as possible to the edge of the cross section of the shank (13), and
the rotation axis extends such that the distance of the rotation axis from the centre of the cross section of the shank (13) of the medical resector (10) decreases strictly monotonically from the proximal end (11) to the distal end (12) of the shank.

2. Medical resector (10) according to the preceding claim, in which the shank (13) has a circular cylindrical barrel surface, and the rotatable radiofrequency electrode (31) comprises a wire loop, of which the centre lies in or on the axis of symmetry of the barrel surface of the shank (13).

3. Medical resector (10) according to one of the preceding claims, also with:
a guide tube (18) in which the shaft (32) is arranged, wherein the guide tube (18) is arranged, at the proximal end (11) of the shank (13), on the outer wall of the shank (13) of the medical resector (10).

4. Medical resector (10) according to one of the preceding claims, also with:
a coupling mechanism (33), at the proximal end (11) of the shank (13), for releasable mechanical coupling to a drive mechanism that provides rotation frequencies in a rotation frequency range contained in the range from 10 revolutions per minute to 200 revolutions per minute.

5. Medical resector (10) according to one of the preceding claims, also with:
a collar (40) for arranging at the distal end (12) of the shank (13) of the medical resector (10), wherein the collar (40) is designed to partially surround the rotatable radiofrequency electrode (31).

6. Medical resector (10) according to the preceding claim, in which the collar (40) has the shape of a cutout of a cylinder jacket.

7. Medical resector (10) according to Claim 5 or 6, in which the collar (40) bears partially on the barrel surface of the shank (13) of the medical resector (10).

8. Medical resector (10) according to one of Claims 5 to 7, in which the collar (40) surrounds the radiofrequency electrode (31) by at least halfway, in relation to the longitudinal axis (19) of the shank (13) of the medical resector (10).

9. Medical resector (10) according to one of Claims 5 to 8, in which the collar (40) surrounds the radiofrequency electrode (31) by at most three-quarters, in relation to the longitudinal axis (19) of the shank (13).

10. Medical resector (10) according to one of Claims 5 to 9, in which a distal end (35) of the radiofrequency electrode (31) protrudes beyond a distal edge (41) of the collar (40).

11. Medical resector (10) according to one of Claims 5 to 9, in which a distal edge (41) of the collar (40) ends flush with a distal end (35) of the radiofrequency electrode (31).

12. Medical resector (10) according to one of Claims 5 to 9, in which a distal edge (41) of the collar (40) protrudes beyond a distal end (35) of the radiofrequency electrode (31).

13. Medical resector (10) according to one of the preceding claims, also with:
a suction opening (54) at the distal end (12) of the medical resector (10), wherein the suction opening (54) is inclined with respect to the longitudinal axis (19) of the medical resector (10).

14. Medical resector (10) according to one of Claims 5 to 12, also with:
a suction opening (54) at the distal end (12) of the medical resector (10), wherein a portion of the edge (55) of the suction opening (54) forms an uninterrupted continuation of a portion of the edge of the collar (40).

## Revendications

1. Dispositif de résection médical (10) pour l'ablation, la découpe ou la coagulation de tissus humains ou animaux, comprenant :
une électrode haute fréquence rotative (31) à l'extrémité distale (12) d'une tige droite (13) du dispositif de résection médical (10) ;
un arbre rigide droit (32) avec un axe de rotation unique droit qui s'étend depuis l'extrémité proximale (11) jusqu'à l'extrémité distale (12) de la tige (13) et qui est accouplé mécaniquement au niveau de l'extrémité distale (12) à l'électrode haute fréquence (31),
l'arbre (32) étant disposé au niveau de l'extrémité distale (12) de la tige (13) au centre ou aussi près que possible du centre de la section transversale de la tige (13) et au niveau de l'extrémité proximale (11) de la tige (13) au bord ou aussi près que possible du bord de la section transversale de la tige (13), et
l'axe de rotation s'étendant de telle sorte que la distance de l'axe de rotation au centre de la section transversale de la tige (13) du dispositif de résection médical (10) diminue de manière strictement monotone depuis l'extrémité proximale (11) jusqu'à l'extrémité distale (12) de la tige.

2. Dispositif de résection médical (10) selon la revendication précédente, dans lequel la tige (13) présente une surface d'enveloppe cylindrique circulaire,
l'électrode haute fréquence rotative (31) comprend une boucle en fil métallique, dont le centre est situé dans ou sur l'axe de symétrie de la surface d'enveloppe de la tige (13).

3. Dispositif de résection médical (10) selon l'une quelconque des revendications précédentes, comprenant en outre :
un tube de guidage (18) dans lequel est disposé l'arbre (32), le tube de guidage (18) étant disposé à l'extrémité proximale (11) de la tige (13) au niveau de la paroi extérieure de la tige (13) du dispositif de résection médical (10).

4. Dispositif de résection médical (10) selon l'une quelconque des revendications précédentes, comprenant en outre un dispositif d'accouplement (33) à l'extrémité proximale (11) de la tige (13), pour l'accouplement mécanique amovible à un dispositif d'entraînement, qui fournit des fréquences de rotation dans une plage de fréquences de rotation comprise entre 10 tr/m et 200 tr/m.

5. Dispositif de résection médical (10) selon l'une quelconque des revendications précédentes, comprenant en outre :
un dispositif de collet (40) destiné à être disposé à l'extrémité distale (12) de la tige (13) du dispositif de résection médical (10), le dispositif de collet (40) étant réalisé pour entourer en partie l'électrode haute fréquence rotative (31).

6. Dispositif de résection médical (10) selon la revendication précédente, dans lequel le dispositif de collet (40) présente la forme d'une portion d'une enveloppe cylindrique.

7. Dispositif de résection médical (10) selon la revendication 5 ou 6, dans lequel le dispositif de collet (40) s'applique en partie contre la surface d'enveloppe de la tige (13) du dispositif de résection médical (10).

8. Dispositif de résection médical (10) selon l'une quelconque des revendications 5 à 7, dans lequel le dispositif de collet (40) entoure au moins la moitié de l'électrode haute fréquence (31) par rapport à l'axe longitudinal (19) de la tige (13) du dispositif de résection médical (10).

9. Dispositif de résection médical (10) selon l'une quelconque des revendications 5 à 8, dans lequel le dispositif de collet (40) entoure au maximum les trois quarts de l'électrode haute fréquence (31) par rapport à l'axe longitudinal (19) de la tige (13).

10. Dispositif de résection médical (10) selon l'une quelconque des revendications 5 à 9, dans lequel une extrémité distale (35) de l'électrode haute fréquence (31) fait saillie par rapport à un bord distal (41) du dispositif de collet (40).

11. Dispositif de résection médical (10) selon l'une quelconque des revendications 5 à 9, dans lequel un bord distal (41) du dispositif de collet (40) se termine en affleurement avec une extrémité distale (35) de l'électrode haute fréquence (31).

12. Dispositif de résection médical (10) selon l'une quelconque des revendications 5 à 9, dans lequel un bord distal (41) du dispositif de collet (40) fait saillie par rapport à une extrémité distale (35) de l'électrode haute fréquence (31).

13. Dispositif de résection médical (10) selon l'une quelconque des revendications précédentes, comprenant en outre :
une ouverture d'aspiration (54) à l'extrémité distale (12) du dispositif de résection médical (10), l'ouverture d'aspiration (54) étant inclinée par rapport à l'axe longitudinal (19) du dispositif de résection médical (10).

14. Dispositif de résection médical (10) selon l'une quelconque des revendications 5 à 12, comprenant en outre :
une ouverture d'aspiration (54) à l'extrémité distale (12) du dispositif de résection médical (10), une portion du bord (55) de l'ouverture d'aspiration (54) formant une prolongation continue d'une portion du bord du dispositif de collet (40).
